# EUROPÄISCHE PATENTSCHRIFT

(11) **EP 4 046 664 B1**
(45) Veröffentlichungstag und Bekanntmachung des Hinweises auf die Patenterteilung: **17.07.2024**
(21) Anmeldenummer: 22154564.3
(22) Anmeldetag: 01.02.2022
(51) Int. Cl.: A61L 9/20

(54) **UV-C-MODUL ZUR RAUMLUFTDESINFEKTION BZW. ENTKEIMUNG VON LUFT DURCH BEHANDLUNG MIT ULTRAVIOLETTER STRAHLUNG**
UV-C MODULE FOR DISINFECTING ROOM AIR STERILISATION OF AIR BY TREATMENT WITH ULTRAVIOLET RADIATION
MODULE UV-C DESTINÉ À LA DÉSINFECTION DE L'AIR AMBIANT OU À LA STÉRILISATION DE L'AIR AU MOYEN DU TRAITEMENT PAR RAYONNEMENT ULTRAVIOLET

(30) Priorität: 04.12.2020 DE 202020107000 U
(43) Veröffentlichungstag der Anmeldung: 24.08.2022
(73) Patentinhaber: biotec Umwelt-Analytik-Beratung-Service GmbH, 33332 Gütersloh (DE); Bermpohl, Andreas, 33332 Gütersloh (DE); Weisser, Jörg, 33332 Gütersloh (DE); Weisser, Frank, 33332 Gütersloh (DE)
(72) Erfinder: Bermpohl, Dr., Andreas, 33332 Gütersloh (DE); Weißer, Jörg, 33332 Gütersloh (DE); Weißer, Frank, 33332 Gütersloh (DE)
(74) Vertreter: Grund, Martin

(56) Entgegenhaltungen:
- EP-A2- 2 455 678
- DE-U1- 202020 106 019
- US-A1- 2005 238 531
- US-B1- 6 746 134

## Beschreibung

### Gebiet der Erfindung

Die Erfindung betrifft ein UV-C-Modul zur Raumluftdesinfektion bzw. Entkeimung von Luft durch Behandlung mit ultravioletter (UV-) Strahlung, sowie ein System, jenes UV-C-Modul umfassend.

### Hintergrund der Erfindung

DE20 2020 106 019 U offenbart ein UV-C-Modul zur Desinfektion bzw. Entkeimung von Luft, die durch RLT-Anlagen umgewälzt, durch Behandlung mit ultravioletter (UV) Strahlung, umfassend mindestens ein Haupt-und ein Stütz-Profil und mindestens einen UV-C-Strahler, wobei das mindestens eine Haupt- und eine Stütz-Profil parallel zueinander angebracht und wobei der mindestens eine UV-C-Strahler über eine Steckverbindung senkrecht zu den Haupt- und Stütz-Profilen angebracht ist und diese miteinander, und wobei der mindestens eine UV-C-Strahler parallel zur Strömungsrichtung der Luft angeordnet ist.

Zentrale Raumlufttechnik- (RLT-) Anlagen bestehen i.d.R. aus zentralen - (RLT-) Geräten mit einem Luftkanalnetz. RLT-Anlagen sind definitionsgemäß mit einer Ventilator gestützten Luftumwälzung ausgestattet, d.h. zentrale RLT-Geräte mit einem Luftkanal oder Luftkanalnetz. RLT-Geräte können Raumluft konditionieren, d.h. erwärmen, kühlen, entfeuchten oder befeuchten. Die Raumluft wird dabei im Umluftbetrieb als Luftstrom in das Gerät angesaugt und nach Konditionierung wieder anteilig oder vollständig über die RLT-Anlage oder das RLT-Gerät in den Raum zurückgeführt, z.B. bei Vorhandensein von RLT-Umluftklappen oder Rotationswärmetauschern (Wärmeräder). RLT-Geräte werden vermehrt in Räumen verwendet, in denen sich dauerhaft Menschen aufhalten, z.B. Büros, Konferenzräume, Unterrichtsräume etc. Durch den Umluftbetrieb solcher Geräte / Anlagen und die damit mechanisch induzierte Luftströmung kann es nun im Raum / Nutzer-Aufenthaltsbereich zu einer Verwirbelung und Verbreitung von Partikeln kommen, die sich in der Raumluft befinden oder in die Raumluft abgegeben werden, etwa durch Sprechen oder Husten. Mitunter kann es sich bei solchen Partikeln um infektiöse, also z.B. bakterielle oder virale Partikel handeln. Der Betrieb von RLT-Geräten / RLT-Anlagen kann somit also die Verbreitung solcher Partikel und damit die Verbreitung von Infektionskrankheiten begünstigen. Gerade in Zeiten eines erhöhten Infektionsgeschehens, siehe auch aktuelle SARS-CoV-2 Pandemie, soll der Umluftbetrieb solcher RLT-Geräte / RLT-Anlagen daher nicht erfolgen.

Da viele Bestands-RLT-Anlagen jedoch planungstechnisch und konstruktiv neben der Außenluft / Frischluftzufuhr u.a. energetisch auf einen Umluftbetrieb ausgelegt sind, kann ein großer Anteil dieser Anlagen nicht ganzjährig ohne Umluftbetrieb betrieben werden. Die Aufrüstung solcher Anlagen mit einem Filter-Upgrade, also einer Erhöhung der Filterklasse ist oft nicht möglich, da dann kein für den Betrieb ausreichender Zuluft-Volumenstrom mehr stattfinden kann. Zudem führt ein so verringerter Luftstrom ebenso zu einer verminderten "Raum-Durchspülung" mit Frischluft / Außenluft und damit zu einem ebenso verringerten Aerosolabtransport aus den versorgten Räumlichkeiten. Ein somit verminderter Zustrom von Frischluft / Außenluft bzw. ein verminderter Luftaustausch konterkariert jedoch die grundsätzliche Empfehlung zum Lüften, d.h. erhöhte Frischluft/ Außenluftzufuhr zur Verdrängung freigesetzter Aerosole.

Die vorliegende Erfindung löst nun dieses Problem, indem es durch das Vor- oder Nachschalten eines sogenannten UV-C-Moduls, eine Möglichkeit zur effektiven Raumluftdesinfektion bzw. - entkeimung bereitstellt, die den Umluftbetrieb der RLT-Geräte auch in Zeiten hoher Infektionsdynamiken erlaubt. UV-C-Module der Erfindung können in bestehende RLT-Geräte einfach nachgerüstet oder auch in neu zu installierende Geräte direkt integriert werden.

### Zusammenfassung der Erfindung

Die vorliegende Erfindung stellt ein UV-C-Modul zur Desinfektion bzw. Entkeimung von Raumluft durch Behandlung mit ultravioletter (UV) Strahlung gemäß Anspruch 1 bereit.

In einer bevorzugten Ausführungsform umfasst das UV-C-Modul mindestens ein Haupt- und ein Stütz-Profil und mindestens einen UV-C-Strahler. Das mindestens eine Haupt- und eine Stütz-Profil sind bevorzugt parallel zueinander angebracht und der mindestens eine UV-C-Strahler über eine Steckverbindung senkrecht dazu und die Profile miteinander verbindend angebracht. Bevorzugt ist der mindestens eine UV-C-Strahler dabei parallel zur Strömungsrichtung der Luft angeordnet.

In einer bevorzugten Ausführungsform ist das mindestens eine Haupt- und Stütz-Profil in y-Ausrichtung angebracht und das Hauptprofil umfasst Steckverbindungen in Form von Lampenfassungen und das Stützprofil Steckverbindungen zum Hindurchstecken oder Ablegen des / der UV-C-Strahler(s).

In einer besonders bevorzugten Ausführungsform ist das mindestens eine Hauptprofil hohl und als Kabelkanal ausgearbeitet.

In einer bevorzugten Ausführungsform umfasst das UV-C-Modul ferner Stabilisierungsprofile zur Stabilisierung der Haupt und Stütz-Profile und UV-C-Strahler in x-, y-, oder z-Ausrichtung.

In einer Ausführungsform sind die Profile aus Aluminium.

In einer bevorzugten Ausführungsform umfassen die Profile höhenverstellbare Stellfüße mit Schraubgewinden.

In einer weiteren bevorzugten Ausführungsform ist der mindestens eine UV-C-Strahler weiterhin von einer Quarzglashülle umgeben.

In einer ganz besonders bevorzugten Ausführungsform ist der mindestens eine UV-C-Strahler weiterhin von einer Teflon-Ummantelung umgeben.

In einer anderen bevorzugten Ausführungsform umfassen die Lampenfassungen Stellringe.

In einer bevorzugten Ausführungsform umfasst die Steckverbindung Keramik, Teflon, UVbeständigen Kunststoff oder Metall.

In einer anderen bevorzugten Ausführungsform ist der mindestens eine UV-C-Strahler weiterhin mit einem UV-C-Sensor verbunden.

Die Erfindung stellt weiterhin ein System zur Aufbereitung und Desinfektion von Luft bereit, die von RLT-Geräten oder -Anlagen umgewälzt wird, wobei das System umfasst:
a) ein Raum-Luft-Technik (RLT) Gerät oder eine RLT Anlage, und
b) ein UV-C-Modul wie hierin offenbart,
wobei das UV-C-Modul autonom ist und modular in das RLT-Gerät oder die RLT-Anlage integriert werden kann.

In einer bevorzugten Ausführungsform des Systems ist das UV-C-Modul im Zuluftkanal oder Abluftkanal des RLT-Geräts oder der RLT-Anlage angebracht, bevorzugt im Zuluftkanal.

In einer anderen Ausführungsform der Erfindung ist das UV-C-Modul eines wie hierin beschrieben und ist mittels verstellbarer Stellfüße und der Stütz-Profile in den Zu- oder Abluftkanal eingespannt.

In einer weiteren Ausführungsform wird das UV-C-Modul über eine Revisionsöffnung in LuftStrömungsrichtung in das RLT-Gerät oder die RLT-Anlage integriert.

In einer weiteren Ausführungsform der Erfindung wird die elektrische Versorgung des UV-C-Moduls durch elektrische Leitungen, die durch den Kabelkanal geführt werden, bereitgestellt.

In einer anderen bevorzugten Ausführungsform umfasst das System ferner eine Steuereinheit, die die UV-C-Strahlerfunktion überwacht.

In einer weiteren bevorzugten Ausführungsform, ist das UV-C-Modul elektrisch so geschaltet, dass es nur dann betrieben werden kann, wenn auch der Ventilator des RLT-Gerätes in Betrieb ist.

In einer anderen Ausführungsform, ist das UV-C-Modul zusätzlich mit einem Kontaktschalter gesichert, der das UV-C-Modul bzw. die UV-C-Strahler nach Öffnung des Moduls automatisch abschaltet.

In einer weiteren bevorzugten Ausführungsform ist das System zusätzlich mit einem Sicherungstürschalter gesichert, so dass die Revisionstür erst nach Betätigung des Not-Aus-Hauptschalters geöffnet werden kann.

### Kurze Beschreibung der Zeichnungen

Fig. 1 stellt schematisch eine Ausführungsform der Erfindung dar. Dargestellt ist ein UV-C-Modul nach einer Ausführungsform der Erfindung, das in den Zuluftkanal einer Bestands-RLT-Anlage integriert ist.
Fig. 2 stellt eine Ausführungsform einer Fassung mit Stellring eines UV-C-Strahlers des UV-C-Moduls der Erfindung dar.
Fig. 3 stellt eine Ausführungsform einer Fassung eines UV-C-Strahlers des UV-C-Moduls der Erfindung dar, eine Kabelverschraubung.

### Ausführliche Beschreibung

### Definitionen

*Desinfizierung bzw. Entkeimung:* Der Begriff Desinfektion bzw. Entkeimung wie hierin verwendet bezieht sich auf die Inaktivierung bzw. Abtötung von Krankheitserregern in Luft um mindestens 99%. Die Begriffe Desinfektion und Entkeimung werden hierhin äquivalent verwendet.

*Krankheitserreger und Keime:* Krankheitserreger wie hierin verwendet bezieht sich auf Mikroorganismen und subzelluläre Erreger, die Krankheiten verursachen können. Hierhin bezieht sich der Begriff vor allem auf Mikroorganismen und subzelluläre Erreger, die Krankheiten in Säugetieren, insbesondere dem Menschen verursachen können. Hierzu gehören vor allem Algen, Bakterien, Parasiten, Pilze, Prionen, Protozoen, Viren und Viroide.

*Profile:* Der Begriff Profil wie hierin verwendet bezieht sich generell auf Rahmen-Konstruktionen, mit rundem oder eckigem Querschnitt. Bevorzugt umfasst ein Profil einen Stab oder eine Stange, der / die je nach Verwendungszeck hohl oder massiv sind kann.

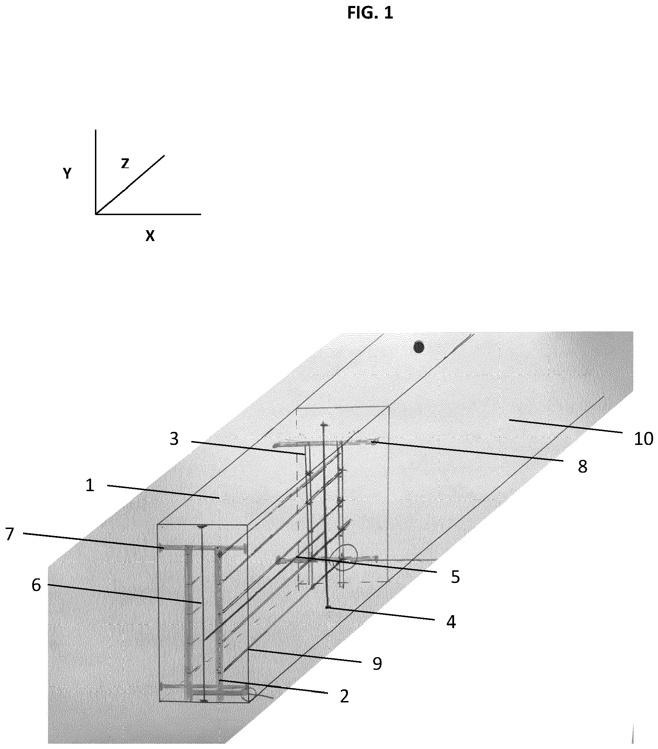

*Hauptprofil:* Der Begriff Hauptprofil wie hierin verwendet bezeichnet eine längliche Konstruktion an der Vorderseite des UV-C-Moduls, die senkrecht zu dem mindestens einen UV-C-Strahler angeordnet ist. Das Hauptprofil ist bevorzugt ein Stab oder eine Stange und hat bevorzugt einen quadratischen oder rechteckigen Querschnitt. Ein Hauptprofil erstreckt sich in y-Ausrichtung und umfasst weiterhin Steckverbindungen und Fassungen für die UV-C-Strahler (s. Fig. 1, (2)). In bevorzugten Ausführungsformen umfasst ein UV-C-Modul mindestens ein Hauptprofil, wobei das Hauptprofil hohl ist und als Kabelkanal ausgestaltet ist, um die in das Hauptprofil eingesetzten UV-C-Strahler mit Strom zu versorgen. Ein UV-C-Modul kann ein, zwei, drei, vier oder mehr Hauptprofile umfassen. Bevorzugt umfasst das UV-C-Modul zwei Hauptprofile.

*Stützprofil:* Der Begriff Stützprofil, wie hierhin verwendet bezeichnet eine dem Hauptprofil gegenüberliegende, längliche Konstruktion des UV-C-Moduls, die senkrecht zu dem mindestens einen UV-C-Strahler angeordnet ist. Das Stützprofil ist bevorzugt ein Stab oder eine Stange und hat bevorzugt einen quadratischen oder rechteckigen Querschnitt. Ein Stützprofil erstreckt sich in y-Ausrichtung und umfasst weiterhin Halterungen für die UV-C-Strahler (s. Fig. 1, (3)). Die Halterungen sind bevorzugt so ausgestaltet, dass die UV-C-Strahler einfach durch das Stützprofil hindurchgesteckt werden können. Alternative können die Halterungen so ausgestaltet sein, dass die UV-C-Strahler darauf abgelegt werden können. Alternativ können die Halterungen als Federklemmen, mit denen die UV-C-Strahler fixiert werden, ausgestaltet sein. Ein UV-C-Modul kann ein oder mehrere Stützprofile umfassen. Die Anzahl der Stützprofile sollte dabei jedoch immer der der Hauptprofile entsprechen. Somit umfasst ein UV-C-Modul bevorzugt zwei Stützprofile.

*Stabilisierungsprofile:* Der Begriff Stabilisierungsprofil wie hierin verwendet bezeichnet eine längliche Konstruktion, die zusätzlich im UV-C-Modul angebracht ist, um die Haupt- und Stützprofile zu stabilisieren. Das Stabilisierungsprofil ist bevorzugt ein Stab oder eine Stange und hat bevorzugt einen runden Querschnitt. Ein Stabilisierungsprofil kann sich in y-Ausrichtung, x-Ausrichtung oder z-Ausrichtung erstrecken. Es kann mittels Steckverbindung oder Hindurchstecken mit Haupt- oder Stützprofilen verbunden sein (s. Fig. 1, (4, 5, 6, 7, 8). Ein UV-C-Modul kann ein, zwei, drei, vier, fünf, sechs, sieben, acht, neun, zehn oder mehr Stabilisierungsprofile umfassen. Bevorzugt umfasst das UV-C-Modul zwei Stabilisierungsprofile, die waagrecht (x-Ausrichtung, s. Fig. 1, (7)) zu den Hauptprofilen angeordnet sind, zwei Stabilisierungsprofile, die waagrecht (x-Ausrichtung, s. Fig. 1, (8)) zu den Stützprofilen angeordnet sind, ein Stabilisierungsprofil, das parallel zu den UV-C-Strahlern angeordnet ist (z-Ausrichtung, s. Fig. 1, (5)) und zwei Stabilisierungsprofile, die senkrecht zu den UV-C-Strahlern angeordnet sind (y-Ausrichtung, s. Fig. 1, (4), (6)).

*UV oder UV-Strahlung:* Die Begriffe UV, UV-Strahlung, UV-C oder UV-C-Strahlung werden hierin äquivalent verwendet. Sie beschreiben elektromagnetische Strahlung im kurzen Wellenlängenbereich. Hierhin wird der Begriff für UV-C-Strahlung im Bereich von um 260 nm verwendet.

*UV-C-Modul und Vorrichtung:* Der Begriff UV-C-Modul wie hierin verwendet beschreibt eine Vorrichtung zur Desinfektion bzw. Entkeimung von Raumluft inklusive ggf. vorhandenem Außenluft- oder Prozessluftanteil, d.h. zur Desinfektion bzw. Entkeimung der der RLT-Anlage zugeführten Luft. Die Begriffe UV-C-Modul und Vorrichtung werden somit äquivalent verwendet.

*UV-C-Strahler und UV-C-Lampen:* Der Begriff UV-C-Strahler wie hierin verwendet beschreibt die UV-C-Quelle, die in einem UV-C-Modul der Erfindung eingesetzt wird um desinfizierende bzw. entkeimende Strahlung zu erzeugen. Die Begriffe UV-C-Strahler und UV-C-Lampe werden äquivalent verwendet.

*x-, y*-, *z-Ausrichtung:* Die Begriffe x-, *y*-, und z-Ausrichtung beschreiben die räumliche Anordnung der Elemente des UV-C-Moduls. x-Ausrichtung beschreibt dabei - wenn die RLT-Anlage von vorn / frontal gesehen wird, die Ausrichtung waagrecht von links nach rechts. y-Ausrichtung beschreibt dabei - wenn die RLT-Anlage von vorn / frontal gesehen wird, die Ausrichtung senkrecht von unten nach oben. z-Ausrichtung beschreibt dabei - wenn dir RLT-Anlage von vorn / frontal gesehen wird - die Ausrichtung von vorn nach hinten in den Raum hinein (s. auch Darstellung in Fig. 1).

### Detaillierte Beschreibung

Die vorliegende Erfindung stellt eine Vorrichtung (UV-C-Modul) bereit, die mittels UV-Strahlung die effektive Desinfektion bzw. Entkeimung von Luft gewährleistet. Sie kann z.B. zusammen mit RLT (Raumlufttechnik) - Geräten/ RLT-Anlagen verwendet werden, um den durch RLT-Geräte / RLT-Anlagen mechanisch erzeugten Luftstrom zu desinfizieren bzw. zu entkeimen. Die vorliegende Erfindung ermöglicht somit den Betrieb von RLT-Geräten / RLT-Anlagen auch dann, wenn zur Vermeidung der Verbreitung von Krankheitserregern, etwa Viren oder Bakterien, die sich in der Raumluft befinden, die mit dem Betrieb von RLT-Geräten / RLT-Anlagen einhergehende Luftströmung eigentlich unerwünscht ist.

Der Wirkungsmechanismus der Desinfektion durch UV-Strahlung beruht dabei auf der photochemischen Veränderung von Teilen der DNA bei Bestrahlung mit Wellenlängen im UV-C-Bereich um 260 Nanometer, insbesondere bei 253,7 nm. Die photochemische Veränderung ist die Dimerisierung benachbarter Thyminbasen an der DNA. Zellen bzw. Mikroorganismen oder subzelluläre Erreger verlieren damit sofort ihre Vermehrungsfähigkeit, es kommt zur Keiminaktivierung.

In der vorliegenden Erfindung wird die UV-Strahlung, die auf die Raumluft einwirken soll, nun durch eine Vorrichtung bereitgestellt. Diese Vorrichtung ist auch als UV-C-Modul bezeichnet. Das UV-C-Modul ist vielseitig einsetzbar und kann unter anderem zur Desinfektion bzw. Entkeimung von Luft in Autos, Bussen, Bahnen, Gondeln, Restaurants, Hotels, Schulgebäuden, Büros, Hallen oder sonstigen räumlich abgeschlossenen Bereichen verwendet werden.

Die folgenden Ausführungsformen sind somit leicht auf sonstige Luftströmungsquellen anwendbar.

Vor allem aber ist das UV-C-Modul für die Reinigung von Luft in Kombination mit RLT-Geräten / RLT-Anlagen vorgesehen. RLT-Geräte / RLT-Anlagen konditionieren Raumluft (ggf. inklusive Außenluft- oder Prozessluftanteil), d.h. sie heizen, kühlen, be- oder entfeuchten Raumluft, um ein angenehmes Innenraumklima zu erzeugen. Dabei wird ein Luftstrom hin zu bzw. weg von den RLT-Geräten / RLT-Anlagen erzeugt. Befinden sich nun Krankheitserreger, Viren, Bakterien oder ähnliches in der (Raum-) Luft, kommt es durch den Betrieb der RLT-Geräte/ RLT-Anlagen zur Verteilung derselben im ganzen Raum. In solchen Fällen wird der Betrieb von RLT-Geräten / RLT-Anlagen daher bislang nicht empfohlen.

Durch das Desinfizieren bzw. Entkeimen des durch RLT-Geräte / RLT-Anlagen erzeugten und umgewälzten Luftstromes kann nun der ungünstige Effekt der Verteilung von Krankheitserregern im ganzen Raum nicht nur minimiert werden, sondern Raumluft sogar gezielt aufbereitet werden. Der Luftstrom, der gegebenenfalls auch Außenluft und Prozessluft enthalten kann, wird dabei einfach zusätzlich durch das UV-C-Modul geleitet und dort desinfiziert bzw. entkeimt, so dass die Luft die RLT-Anlagen- / UV-C-Modul-Kombination oder RLT-Gerät- / UV-C-Modul-Kombination konditioniert und nahezu keimfrei verlässt.

Das UV-C-Modul der Erfindung kann einfach in bestehende RLT-Anlagen nachgerüstet werden. Die Montage des UV-C-Moduls erfolgt dazu einfach im Zuluftkanal oder Ab-/ Umluftkanal der Anlage. Das UV-C-Modul ist ein Montage-Stecksystem aus Einzelprofilen, das somit selbst auf kleinem Raum einfach und individuell in die Anlage integriert bzw. eingebaut werden kann. Zur Installation werden dabei lediglich eine Öffnung, eine sogenannte Revisionsöffnung, und ein kleiner Durchlass zur Kabelführung auf einer Seite der RLT-Anlage, z.B. in einem frei wählbaren zugänglichen Bereich des RLT-Zuluftkanals oder des RLT-Ab- oder - Umluftkanals benötigt. Durch diese Revisionsöffnung erfolgt die Einbringung des im Kanal zu montierenden Materials, d.h. des UV-C-Moduls. Die Montage des Moduls erfolgt im Kanal, der Monteur befindet sich jedoch während der Montage stets außerhalb der RLT-Anlage.

Durch die einfache Montage und die Möglichkeit zur Integration in bereits bestehende RLT-Anlagen bietet das UV-C-Modul der Erfindung signifikante Vorteile gegenüber bereits bestehenden Lösungen zur Raumluftdesinfektion.

Bisher verfügbare Lösungen erfordern etwa die mit hohem Montageaufwand verbundene komplette Deinstallation eines Kanalteilabschnitts aus dem RLT-Luftleitungssystem und daraufhin den Einbau eines neuen mit UV-C-Lampen versehenen Kanalabschnitts. In der Praxis ist eine derartige Nachrüstung aufgrund der i.d.R. beengten Verhältnisse in den Technikzentralen besonders aufwändig.

Weiterhin sind gut zugängliche Bereiche des zentralen RLT-Zuluftkanals häufig bereits mit Komponenten wie Schalldämpfern u.a. verbaut und stehen somit zur Installation von UV-C-Nachrüstungen nicht zur Verfügung. Hinzu kommt, dass die Verzweigung der Luft-Kanalführung i.d.R. bereits in der Technikzentrale oder kurz danach beginnt, so dass eine zentrale Installation eines UV-C-Moduls in diesen Fällen nur in der Technikzentrale selbst möglich ist. Dezentrale Montagen von möglichen UV-C-Anwendungen sind ebenfalls problematisch, da die Lüftungskanäle im Gebäude meist in Zwischendecken verlaufen mit entsprechend beengten Platzverhältnissen und erschwerter Zugänglichkeit. Mehrere dezentrale UV-Anwendungen sind zudem wiederum kostenintensiver als ein zentrales UV-C-Modul.

Im Gegensatz zu bisher verfügbaren Lösungen bietet das UV-C-Modul der Erfindung ein flexibles, individuell anpassbares und jederzeit erweiterbares modulares Stecksystem, das die Demontage bereits bestehender Anlagen oder Teilen davon nicht erfordert. Das Bausatzprinzip aus Einzelkomponenten des UV-C-Moduls der vorliegenden Erfindung ermöglicht weiterhin die Materialeinbringung und den Aufbau des Moduls selbst in beengten Räumen.

Genauso ist das UV-C-Modul jederzeit frei revisionierbar, d.h. es kann jederzeit einfach vollständig demontiert werden (Stichwort: Plug & Play), z.B. um die Anlage zu reinigen oder sonstige Instandhaltungsarbeiten durchzuführen. Die Montage des gesamten UV-C-Moduls erfolgt selbsttragend, d.h. es sind keine Montagebefestigungen wie Bohrungen oder Halterungen notwendig.

Zusätzlich sind elektrische Anschlüsse als Steckerverbindungen ausgeführt, um eine einfache Montage/ Demontage zu ermöglichen.

Alle Bauteile, die im RLT-Luft-Kanal montiert werden, d.h. nahezu alle Bestandteile des UV-C-Moduls, die nicht UV-C-Strahler sind, bestehen vorzugsweise aus Leichtbaumaterialen, wie Aluminium, die zugleich korrosions- und UV-beständig sind.

UV-C-empfindliche Materialien, wie Kabel, werden geschützt im Kabelkanal aus dem RLT-Kanal abgeführt.

Die UV-C-Strahler, die in UV-C-Modulen der vorliegenden Erfindung verbaut werden, werden in Längsrichtung, d.h. in Richtung des Luftstromes, den es zu desinfizieren bzw. zu entkeimen gilt, ausgerichtet. Die Luft erfährt somit eine optimierte erhöhte Bestrahlungszeit und -dosis, d.h. Krankheitserreger im Luftstrom sind entlang der Strahler-Länge einer höheren Bestrahlungszeit und -dosis ausgesetzt als wenn die Strahler quer zur Luftströmungsrichtung angeordnet sind, wie dies bei einigen bisherigen UV-C-Lösungen der Fall ist. Solche bisher verfügbaren UV-C-Lösungen, die auf eine Querausrichtung der Strahler setzen, bergen zudem oft das Problem, dass bei bestehenden RLT-Anlagen häufig nicht ausreichend Kanalstrecke von außen zugänglich ist, um den Einbau der Anzahl von Strahler durchzuführen, die für eine effektive Luftreinigung nötig wäre.

Die Längsausrichtung der UV-C-Strahler hat in der Praxis zudem den Vorteil, dass RLT-Anlagen unabhängig vom Kanalquerschnitt, der zwischen verschiedenen Anlagen stark variieren kann, mit UV-C-Strahlern mit definierten und standardisierten Längen und Größen ausgestattet werden können. Auch der Abstand der Strahler zueinander kann standardisiert bzw. im Bedarfsfall frei festgelegt werden. Da die Bestrahlungsdosis im Quadrat zum Abstand vom UV-C-Strahler abnimmt (doppelter Abstand bedeutet nur noch 25% Strahlungsdosis) kommt der gleichmäßigen räumlichen Anordnung der UV-C-Strahler im Luftkanal besondere Bedeutung zu, um nicht nur eine nicht-ausreichende Bestrahlung und damit unzureichende Inaktivierung von Krankheitserregern zu vermeiden, sondern auch um Resistenz-verleihende Mutationen der Krankheitserreger zu vermeiden. Somit kann das System zum einen kosteneffektiv, zum anderen standardisiert gehalten werden und damit sichergestellt werden, dass eine ausreichende Bestrahlung und Desinfektion bzw. Entkeimung stattfindet.

Schließlich wird eine bestrahlungsmindernde Beschattung der zu reinigenden Luft durch im Luftstrom enthaltene Staubpartikel verhindert, indem das UV-C-Modul bevorzugt im Zuluftkanal integriert ist. Die Luft passiert somit erst nach erfolgter obligatorischer Filterung im RLT-Zentralgerät die UV-C-Strahler und erfährt somit eine optimale UV-C-Bestrahlung.

Ein UV-C-Modul der Erfindung umfasst bevorzugt mindestens ein Hauptprofil, ein Stützprofil und mindestens einen UV-C-Strahler. In einer Ausführungsform sind das mindestens eine Hauptprofil und das mindestens eine Stützprofil parallel zueinander angeordnet und der mindestens eine UV-C-Strahler senkrecht zu den Profilen so angeordnet, dass er diese über Steckverbindungen in z-Ausrichtung miteinander verbindet. Der mindestens eine UV-C-Strahler ist dabei in die Strahlerfassung, die vom Hauptprofil umfasst ist, eingesteckt und liegt mit seinem Ende in der Halterung, die vom Stützprofil umfasst ist. Die Haupt- und Stütz-Profile werden parallel zu zwei der Kanalwände und senkrecht zu den beiden anderen Kanalwänden in den Luftkanal eingebracht. Das UV-C-Modul kann ferner Stabilisierungsprofile umfassen, die jeweils parallel oder senkrecht zueinander wie auch zu den Haupt- und Stützprofilen in x-, y-, oder z-Ausrichtung angebracht sind. Die Profile ergeben miteinander verbunden eine dreidimensionale Gitterstruktur.

Ein UV-C-Strahler kann ein Niederdruckstrahler, Mitteldruckstrahler oder ein UV-LED-Strahler sein, bevorzugt ein Niederdruckstrahler.

In einer bevorzugten Ausführungsform ist ein UV-C-Strahler als UV-C-Doppelstrahler ausgebildet. Bevorzugt können die Haupt-, Stütz- und Stabilisierungsprofile aus Aluminium bestehen.

An den oberen und seitlichen Enden der Profile können Stellfüße angebracht sein. Bevorzugt sind die Stellfüße über Schraubgewinde höhenverstellbar. Bevorzugt sind sie Stellfüße an den Profilen vormontiert und werden nach Einbringen des Profils in den Luftkanal so aufgedreht, dass das Profil fest mit der Kanalwand verbunden ist. Das Festsitzen der Profile in dem Kanal kann zusätzlich mit Kontermuttern abgesichert werden.

In einer bevorzugten Ausführungsform umfassen die Hauptprofile einen Kabelkanal. Der Kabelkanal kann somit die Stromversorgung, die zum Betrieb der UV-C-Strahler nötig ist, bereitstellen. Der Kabelkanal kann dabei selbst wesentlich als Hauptprofil dienen und die Stützfunktion des UV-C-Moduls ausüben, indem am Ende des Kabelkanals ein Stellfuß montiert ist, der zur Fixierung des Kabelkanals in der Kanalwand ausgedreht wird, wie dies auch bei "normalen" Rechteck-Profilen der Fall ist. Der Kabelkanal ist bevorzugt an einer der Längsseiten zu öffnen bzw. revisionierbar, z.B. über Steckschienen, Schienen mit einem Blenden-Schnapp- / Klickmechanismus oder aufklappbar. Die Kabel der UV-C-Lampen werden gebündelt und über den Kabelkanal, zunächst senkrecht und dann seitlich aus dem RLT-Kanal, durch eine Öffnung in der Kanalwand, nach außen geführt (bevorzugt im Bodenbereich des RLT-Kanals).

Für größere Anlagen können die Profile mit Teleskopprofilen ausgestattet sein. Die Montage kann über eine vordere Öffnung/ Revisionsöffnung erfolgen, d.h. im Bereich der Vorderseite des Luftkanals, und - falls erforderlich - über zusätzlich eine hintere Öffnung / Revisionsöffnung, d.h. auf der Rückseite des Luftkanals.

Das UV-C-Modul wird bevorzugt elektrisch so geschaltet, dass es nur dann betrieben werden kann, wenn auch der Ventilator des RLT-Gerätes in Betrieb ist. Zur Sicherung kann das UV-C-Modul zusätzlich mit einem Kontaktschalter gesichert sein, so dass beim Öffnen des UV-C-Moduls der / die UV-C-Strahler automatisch abgeschaltet werden. In einer weiterhin bevorzugten Ausführung kann über einen elektrisch angebundenen Sicherungstürschalter realisiert werden, dass die Revisionstür nur nach Aktivierung / Betätigung des elektrischen Not-Aus-Hauptschalters geöffnet werden kann.

Bevorzugt wird der mindestens eine UV-C-Strahler über Steckverbindungen mit den Haupt- und Stütz-Profilen verbunden. In den Hauptprofilen sind die Steckverbindungen bevorzugt als ringförmiges Loch in den Profilen ausgebildet, in die die Fassungen der UV-C-Strahler geschoben werden können, die zugleich als Halter für die UV-C-Strahler dienen. Dafür können Fassungen, bevorzugt aus Keramik am Hauptprofil vorgesehen sein. Die Fassungen können ebenfalls aus Teflon oder anderen Materialien bestehen die zugleich UV-beständig sind.

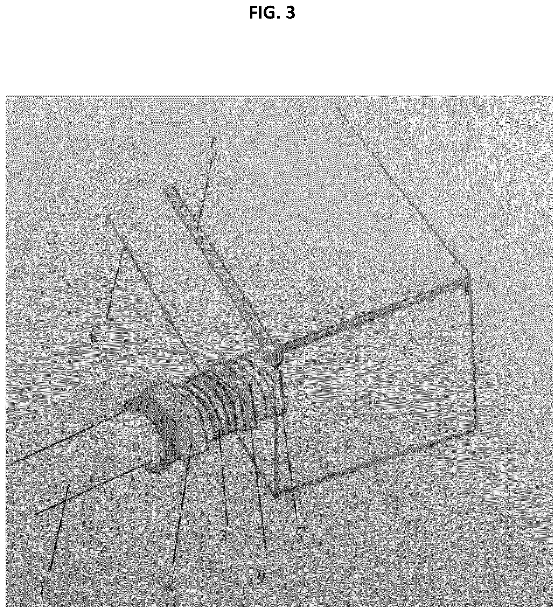

In einer anderen besonders bevorzugten Ausführungsform sind die Fassungen der Hauptprofile als Kabelverschraubungen ausgestaltet. Kabelverschraubungen bestehen bevorzugt aus Edelstahl. Ebenfalls umfassen Kabelverschraubungen bevorzugt eine Quetschdichtung. In einer Ausführungsform werden die UV-C-Strahler somit über Kabelverschraubungen aus Edelstahl mit innenliegender Quetschdichtung fixiert (siehe Fig. 3). Die Quetschdichtung kann dabei durch Anziehen einer äußeren Schraubgewindemutter an den UV-C-Strahler-Durchmesser angepasst werden und so den UV-C-Strahler weich und elastisch umgeben. Die Kabelverschraubung, welche senkrecht durch die Profilwand durch eine Bohrung integriert wird, besteht aus einem kurzen Rohr mit Außengewinde, auf dem die Muttern aufgeschraubt werden. Im Rohr befindet sich die Quetschdichtung. Die Strahler-Fassung wird montageseitig in die Kabelverschraubung innenliegend integriert. Auf der innenliegenden Seite des Profils / Kabelkanals wird die Fixierung der Kabelverschraubung am Profil durch ein Anziehen der inneren Gegenmutter realisiert.

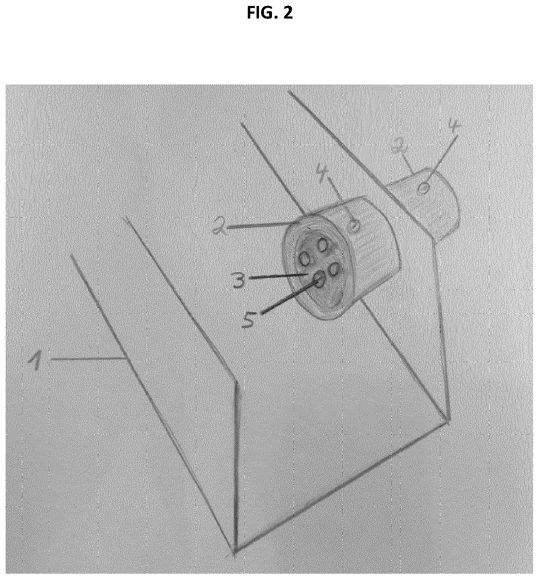

Alternativ können die Fassungen bevorzugt mit zwei Stellringen / Stellverbindungen in das Profil integriert werden (Fig. 2). Hierbei ist jeweils ein Stellring auf der einen Seite des Profils (außen) und ein Stellring auf der anderen Seite des Profils (innen) über die Fassung gestülpt / durchgesteckt. Durch Festdrehen der in den Stellringen integrierten Inbusschrauben erfolgt die Fixierung der Stellringe an der Fassung und die Fixierung im / am Profil. In die Fassungen werden die UV-Lampen als elektrischer Anschluss eingesteckt.

In den Stützprofilen sind die Steckverbindungen bevorzugt als ringförmige Löcher in den Profilen ausgebildet, in die das andere Ende der UV-Lampen hindurchgesteckt oder abgelegt werden kann. Die Halterungen können aus Teflon sein oder anderen Materialien, die keine Beschädigungen (z.B. Kratzspuren) auf den UV-C-Strahlern hinterlassen. Die UV-C-Strahler können statt in einer Halterung in den Stützprofilen auch über eine Federklemme, einen Metallclips oder eine Metallspange außen am Profil befestigt sein.

Der UV-C-Strahler kann auch von einem Quarzglas-Hüllrohr umgeben sein, wodurch ein zusätzlicher mechanischer Schutz gewährleistet wird. Das Quarzglashüllrohr kann am Ende geschlossen oder offen sein. Das Quarzglas-Hüllrohr ist für UV-C-Strahlung durchlässig. In einer bevorzugten Ausführungsform ist der UV-C-Strahler mit einem Quarzglas-Hüllrohr umgeben und über ein Gewindeübergangsstück inkl. Dichtungsring mit dem als Kabelkanal ausgebildeten Rechteck-Profil verbunden.

In einer weiteren Ausführung können die UV-C-Strahler mit einer Teflon (ETFE, FEP) - Ummantelung ausgestattet sein. Eine Teflon-Ummantelung schützt mechanisch vor Beschädigungen und ggf. auftretender Feuchtigkeit. In Bereichen mit stark gekühltem Luftstrom (z.B. in der Fleischwarenindustrie) verhindert sie einen Leistungsabfall der UV-C-Strahler, da die UV-C-Leistung der UV-C-Strahler bei entsprechender Abkühlung der Umgebungstemperatur sinkt.

Die UV-C-Strahler werden vorzugsweise über elektronische Vorschaltgeräte (EVG) betrieben, um einen schnellen und flackerfreien Start der UV-C-Strahler zu gewährleisten. Zielführend sind EVG's, welche mehrere UV-C-Strahler versorgen können.

Die Funktionsfähigkeit der UV-C-Strahler kann zusätzlich durch UV-C-Sensoren überwacht werden. In einer einfachen Ausführung wird der Strahlerwechsel über einen Betriebsstundenzähler und/oder über die elektrische Stromstärke (Stromfluss) angezeigt.

In einer bevorzugten Ausführung können die UV-C-Strahler mit einer externen Steuereinheit zur Überwachung der UV-C-Strahlerfunktion versehen werden. Hierzu ist ferner auch eine Aufschaltung der Steuerungssignale auf die entsprechende Gebäudeleittechnik zur Fehlermeldung und Überwachung der Betriebsparameter praktikabel.

### Ausführungsbeispiele

Ausführungsbeispiele der hierhin beschriebenen Erfindung werden in Fig. 1 und 2 dargestellt.

Figur 1 zeigt die schematische Darstellung eines UV-C-Moduls (1) zur Raumluftdesinfektion bzw. Entkeimung durch Behandlung mit ultravioletter Strahlung. Das Modul (1) umfasst dabei mindestens zwei Hauptprofile (2) und mindestens einen UV-C-Strahler (9). Das Modul ist vorzugsweise in ein RLT-Gerät/ den Zuluftkanal einer RLT-Anlage (10) integriert.

In Fig. 1 sind die mindestens zwei Hauptprofile als Kabelkanal (2) ausgestaltet, der die Stromversorgung des / der UV-C-Strahler(s) bereitstellt. Ebenfalls umfasst das UV-C-Modul zwei Stützprofile (3), sieben Stabilisierungsprofile ((4), (5), (6), (7) und (8)).

Fig. 2 zeigt ein Schema eines UV-C-Moduls mit einem Stellring nach einer Ausführungsform der Erfindung. Dabei werden die Fassungen für UV-C-Strahler über Stellringe (2) in das Profil (1), das auch als Kabelkanal ausgestaltet sein kann, integriert. Hierbei ist jeweils ein Stellring auf der einen Seite des Profils (außen) und ein Stellring auf der anderen Seite des Profils (innen) über die Fassung (3) gestülpt / durchgesteckt. Durch Festdrehen der in den Stellringen integrierten Inbusschrauben (4) erfolgt die Fixierung der Stellringe an der Fassung und die Fixierung im / am Profil. In die Fassungen werden die UV-C-Strahler als elektrischer Anschluss eingesteckt (5).

Fig. 3 zeigt ein Schema einer Kabelverschraubung zur Fixierung von UV-C-Strahlern an Hauptprofilen, die auch als Kabelkanal ausgestaltet sein können, im UV-C-Modul. Dazu ist die Kabelverschraubung am Hauptprofil / Kabelkanal (6) angebracht. Die Kabelverschraubung umfasst bevorzugt die folgenden Komponenten: (2) äußere Mutter mit innenliegender Quetschdichtung, (3) Gewinde, (4) äußere Gegenmutter, und (5) innere Gegenmutter. Der UV-C-Strahler (1) kann durch die Anpassung der Quetschdichtung sicher, aber elastisch in der Verschraubung fixiert werden. Das Hauptprofil / der Kabelkanal wird nach außen hin durch eine Abdeckung des Kabelkanals, auch Deckschiene genannt (7), abgeschlossen.

## Patentansprüche

1. UV-C-Modul zur Desinfektion bzw. Entkeimung von Luft, die durch Raumlufttechnik (RLT)-Anlagen umgewälzt wird, durch Behandlung mit ultravioletter (UV) Strahlung, umfassend mindestens ein Haupt- und ein Stütz-Profil (2, 3) und mindestens einen UV-C-Strahler, (9)
wobei das mindestens eine Haupt- und eine Stütz-Profil parallel zueinander angebracht sind, wobei jenes mindestens eine Haupt- und eine Stütz-Profil eine geradlinige Erstreckung aufweisen, wobei das Hauptprofil hohl ist und als Kabelkanal (2) ausgearbeitet ist und
wobei der mindestens eine UV-C-Strahler (9) parallel zur Strömungsrichtung der Luft angeordnet ist, und wobei der mindestens eine UV-C-Strahler (9) über eine Steckverbindung senkrecht zu den Haupt- und Stütz-Profilen angebracht ist und diese miteinander verbindet, und wobei das UV-C-Modul im Zuluftkanal oder Abluftkanal (2, 3) der RLT-Anlage angebracht ist.

2. Das UV-C-Modul zur Desinfektion bzw. Entkeimung Luft nach Anspruch 1, wobei das Haupt-und Stütz-Profil in einer y-Ausrichtung angebracht sind und das Hauptprofil Steckverbindungen in Form von Lampenfassungen umfasst und das Stütz-Profil Steckverbindungen zum Hindurchstecken oder Ablegen des / der senkrecht dazu angeordneten UV-C-Strahler(s) gewährleistet.

3. Das UV-C-Modul zur Desinfektion bzw. Entkeimung von Luft nach einem der vorhergehenden Ansprüche, wobei das UV-C-Modul ferner Stabilisierungsprofile zur Stabilisierung der Haupt- und Stütz-Profile und UV-C-Strahler in x-, y-, oder z-Ausrichtung umfasst.

4. Das UV-C-Modul zur Desinfektion bzw. Entkeimung von Luft nach einem der vorhergehenden Ansprüche, wobei die Profile aus Aluminium sind und/ oder höhenverstellbare Stellfüße mit Schraubgewinden umfassen.

5. Das UV-C-Modul zur Desinfektion bzw. Entkeimung von Luft nach einem der vorhergehenden Ansprüche, wobei der UV-C-Strahler weiterhin von einer Quarzglashülle und/ oder einer Teflon-Ummantelung umgeben ist.

6. Das UV-C-Modul zur Desinfektion bzw. Entkeimung von Luft nach einem der vorhergehenden Ansprüche, wobei die Lampenfassungen Stellringe umfassen.

7. Das UV-C-Modul zur Desinfektion bzw. Entkeimung von Luft nach einem der vorhergehenden Ansprüche, wobei die Steckverbindung Keramik, Teflon, UVbeständigen Kunststoff oder Metall umfasst.

8. Das UV-C-Modul zur Desinfektion bzw. Entkeimung von Luft nach einem der vorhergehenden Ansprüche, wobei der UV-C-Strahler weiterhin mit einem UV-C-Sensor verbunden ist.

## Claims

1. UV-C module for disinfecting or sterilizing air which is circulated by ventilation and air-conditioning (HVAC) systems by treatment with ultraviolet (UV) radiation, comprising at least one main profile (2) and one support profile (3) and at least one UV-C emitter (9), wherein the at least one main profile and one support profile are mounted parallel to each other, wherein said at least one main profile and one support profile have a rectilinear extension, wherein the main profile is hollow and is designed as a cable duct (2) and
wherein the at least one UV-C emitter (9) is mounted parallel to the air flow direction and,
wherein the at least one UV-C emitter (9) is mounted perpendicular to the main and support profiles via a plug-in connection which connects them to one another, and wherein the UV-C module is mounted in the supply air duct or exhaust air duct (2,3) of the ventilation and air conditioning system.

2. UV-C module for disinfecting or sterilizing air according to claim 1, wherein the main and support profiles are mounted in a y-alignment and the main profile comprises plug-in connections in the form of lamp sockets and the support profile ensures plug-in connections for inserting through or depositing the UV-C emitter(s) arranged perpendicularly thereto.

3. UV-C module for disinfecting or sterilizing air according to one of the preceding claims, wherein the UV-C module further comprises stabilizing profiles for stabilizing the main and support profiles and UV-C emitters in x, y or z orientation.

4. UV-C module for the disinfection or sterilization of air according to one of the preceding claims, wherein the profiles are made of aluminum and/or comprise height-adjustable adjustable feet with screw threads.

5. UV-C module for disinfecting or sterilizing air according to one of the preceding claims, wherein the UV-C emitter is further surrounded by a quartz glass envelope and/or a Teflon sheath.

6. UV-C module for disinfecting or sterilizing air according to one of the preceding claims, wherein the lamp sockets comprise adjusting rings.

7. UV-C module for disinfecting or sterilizing air according to one of the preceding claims, wherein the plug-in connection comprises ceramic, Teflon, UV-resistant plastic or metal.

8. UV-C module for disinfecting or sterilizing air according to one of the preceding claims, wherein the UV-C emitter is further connected to a UV-C sensor.

## Revendications

1. Module UV-C de désinfection ou de stérilisation de l'air, qui circule dans des installations techniques de ventilation de locaux, par traitement avec un rayonnement ultraviolet (UV),
comprenant au moins un profilé principal et un profilé de support (2, 3) et au moins un émetteur UV-C (9),
dans lequel l'au moins un profilé principal et l'au moins un profilé de support sont installés de manière parallèle l'un par rapport à l'autre, dans lequel l'au moins un profilé principal et l'au moins un profilé de support présentent une extension rectiligne, dans lequel le profilé principal est creux et est conçu en tant que goulotte pour câble (2), et
dans lequel l'au moins un émetteur UV-C (9) est disposé de manière parallèle à la direction d'écoulement de l'air, et
dans lequel l'au moins un émetteur UV-C (9) est installé de manière perpendiculaire aux profilés principaux et de support par l'intermédiaire d'une liaison par enfichage et relie ceux-ci l'un à l'autre, et
dans lequel le module UV-C est installé dans le canal d'air entrant ou le canal d'air sortant (2, 3) de l'installation technique de ventilation de locaux.

2. Module UV-C de désinfection ou de stérilisation de l'air selon la revendication 1, dans lequel le profilé principal et le profilé de support sont installés dans une orientation y et le profilé principal comprend des liaisons par enfichage sous la forme de douilles de lampe et le profilé de support assure des liaisons par enfichage pour insérer par enfichage ou déposer l'émetteur/les émetteurs UV-C disposés de manière perpendiculaire à celui-ci.

3. Module UV-C de désinfection ou de stérilisation de l'air selon l'une quelconque des revendications précédentes, dans lequel le module UV-C comprend en outre des profils de stabilisation pour stabiliser les profilés principaux et de support et des émetteurs UV-C dans l'orientation x, y ou z.

4. Module UV-C de désinfection ou de stérilisation de l'air selon l'une quelconque des revendications précédentes, dans lequel les profilés sont en aluminium et/ou comprennent des pieds de réglage ajustables en hauteur avec des pas de vis.

5. Module UV-C de désinfection ou de stérilisation de l'air selon l'une quelconque des revendications précédentes, dans lequel l'émetteur UV-C est entouré par ailleurs d'une coque en verre de quartz et/ou d'une coque en Téflon.

6. Module UV-C de désinfection ou de stérilisation de l'air selon l'une quelconque des revendications précédentes, dans lequel les douilles de lampe comprennent des bagues de réglage.

7. Module UV-C de désinfection ou de stérilisation de l'air selon l'une quelconque des revendications précédentes, dans lequel la liaison enfichable comprend de la céramique, du Téflon, une matière plastique résistante aux UV ou du métal.

8. Module UV-C de désinfection ou de stérilisation de l'air selon l'une quelconque des revendications précédentes, dans lequel l'émetteur UV-C est relié par ailleurs à un capteur UV-C.
